# FASCICULE DE BREVET EUROPEEN

(11) **EP 0 858 329 B1**
(45) Date de publication et mention de la délivrance du brevet: **04.06.2003**
(21) Numéro de dépôt: 97936739.8
(22) Date de dépôt: 05.08.1997
(51) Int. Cl.: A61K 31/165, A61K 47/12, A61K 47/20, A61K 47/02, A61K 47/26

(54) **NOUVELLES FORMULATIONS LIQUIDES STABLES A BASE DE PARACETAMOL ET LEUR MODE DE PREPARATION**
NEUE STABILE FLÜSSIGE PARACETAMOLMITTEL UND VERFAHREN ZU DEREN HERSTELLUNG
NOVEL STABLE LIQUID PARACETAMOL COMPOSITIONS, AND METHOD FOR PREPARING SAME

(30) Priorité: 05.08.1996 FR 9609858
(43) Date de publication de la demande: 19.08.1998
(73) Titulaire: SCR Pharmatop, 78000 Versailles (FR)
(72) Inventeur: DIETLIN, François, F-78230 Le Pecq (FR); FREDJ, Danièle, F-91190 Gis sur Yvette (FR)
(74) Mandataire: Burtin, Jean-François
(86) Numéro de dépôt international: FR9701452
(87) Numéro de publication internationale: WO98005314

(56) Documents cités:
- WO-A-95/23595
- DE-A- 4 327 462
- US-A- 4 314 989
- US-A- 5 474 757
- CHEMICAL ABSTRACTS, vol. 105, no. 26, 29 décembre 1986 Columbus, Ohio, US; abstract no. 232386, YAN, ZHENG; ET AL.: "Preparation of paracetamol injections" XP002030816 & YAOXUE TONGBAO, vol. 21, no. 7, 1986, pages 387-389,
- DATABASE WPI Week 9444 16 août 1991 Derwent Publications Ltd., London, GB; AN 94-355463 XP002030817 & KR 9 311 994 A (DAEKWANG PHARM. CO.) , 23 décembre 1993
- DATABASE WPI Week 8526 Derwent Publications Ltd., London, GB; AN 85-154902 XP002045739 & HU 34 687 A (EGYT GYOGYSZERVEGYESZETI GYAR) , 29 avril 1985
- DATABASE WPI Week 8423 Derwent Publications Ltd., London, GB; AN 84-144236 XP002045740 & RO 82 841 A (INTR MEDICAMENTE BIOFARM) , 30 octobre 1983
- CHEMICAL ABSTRACTS, vol. 123, no. 20, 13 novembre 1995 Columbus, Ohio, US; abstract no. 266132, XP002045737 & JP 07 206 689 A (BAANETSUTO LAB LTD.) 8 août 1995

## Description

La présente invention concerne de nouvelles formulations antalgiques liquides, stables. à base de paracétamol, associé ou non à un dérivé analgésique.

Il est connu, depuis de nombreuses années et notamment par un article de FAIRBROTHE J.E.. intitulé : Acetaminophen, paru dans Analytical Profiles of Drug Substances (1974), volume 3, Pages 1-109, que le paracétamol placé en milieu humide, et « a fortiori » également lorsqu'il se trouve en solution aqueuse, est susceptible de subir une hydrolyse pour former du p-aminophénol, lui-même susceptible de se dégrader en quinoneimine. La vitesse de dégradation du paracétamol croît avec l'augmentation de la température et à la lumière.

Par ailleurs, on a déjà largement décrit l'instabilité du paracétamol en solution aqueuse en fonction du pH de la solution. Ainsi, selon l'article « Stability of aqueous solutions of N-acetyl-p-aminophenol » (KOSHY K.T. et LACH J.l. J. Pharm. Sci., 50, (1961), pages 113-118), le paracétamol en solution aqueuse présente une instabilité qui se traduit par, en premier lieu, une hydrolyse aussi bien en milieu acide qu'en milieu alcalin. Cette dégradation est minimale à un pH voisin de 6, la demi-vie de dégradation atteignant dans ce cas 21,8 années à 25°C.

L'application de la loi d'Arrhenius à l'aide de la constante de réaction spécifique déterminée par ces auteurs conduit à calculer un temps d'environ 19 mois pour observer une baisse de 5 % du titre en paracétamol d'une solution aqueuse conservée à 25°C au pH optimum. Indépendamment de l'hydrolyse, la molécule de paracétamol subit un autre type de décomposition par formation d'une quinone-imine susceptible de se polymériser en donnant naissance à des polymères azotés.

Ces polymères et notamment ceux de N-acétyl p-benzoquinone-imine ont été décrits en outre comme étant le métabolite toxique du paracétamol, notamment cytotoxique et hémolytique. La décomposition de ce métabolite en milieu aqueux est encore plus complexe et donne naissance à de la p-benzoquinone et à de l'hydroquinone (D.DAHLIN J.Med Chem. 25 (1982) 885-886).

Dans l'état actuel de l'art et compte tenu des exigences de qualité propres à la réglementation pharmaceutique, la stabilité du paracétamol en solution aqueuse est de ce fait insuffisante et ne permet pas la réalisation de compositions pharmaceutiques liquides injectables. En conséquence la mise au point de formes pharmaceutiques liquides, notamment injectables, de paracétamol, était restée sans solution.
Certains essais ont été réalisés afin de limiter la dégradation du paracétamol en solution aqueuse. Ainsi, dans un article intitulé : Stabilisation by éthylènediamine tétraacetic acid of amid and other groups in drug compound, (FOGG Q.G et SUMMAN A.M, J. Clin. Pharm. Ther, 17, (1992) 107-109), il est indiqué qu'une solution aqueuse de paracétamol à 0,19% présente un taux de p-aminophénol, produit d'hydrolyse du paracétamol, qui atteint 19,8% du taux initial de paracétamol après conservation à l'obscurité pendant 120 jours. L'addition d'EDTA à raison de 0,0075%, limite cette dégradation à 7%. Par ailleurs, la distillation d'une solution alcaline de paracétamol engendre une teneur de 14% en ammoniaque, en présence ou non de 1000 ppm d'acide ascorbique. En effet, l'acide ascorbique présente des propriétés satisfaisantes pour une telle stabilisation. Cependant, exposée à une lumière intense, une solution de paracétamol contenant 1000 ppm d'acide ascorbique produit malgré cela de l'ammoniaque avec un rendement de 98%. En revanche, l'addition d'EDTA (0,0075%) à cette solution limite la dégradation, le rendement en ammoniaque n'excédant pas 14%.

La référence Yan, Yaoxue Tongbao (1986) 21 (7) 387-389 décrit l'utilisation de bisulfite de sodium comme antioxydant dans des solutions de paracétamol dans le poly éthylène glycol. Cette référence est limitée à l'emploi de bisulfite pour essayer d'obtenir la stabilité de la solution de paracétamol.

II faut retenir en particulier l'indication fournie dans cette publication selon laquelle la présence d'un agent antioxydant n'a pas un effet significatif sur la stabilité du paracétamol mais peut éviter la coloration de la solution.

En dépit de toutes ces tentatives, on n'avait pas pu préparer des solutions liquides aqueuses de paracétamol et notamment des solutions injectables, dont la stabilité puisse être garantie.

La présente invention a pour objet de résoudre ce problème d'une manière commode et satisfaisante. Elle concerne des compositions pharmaceutiques libres stables à l'oxydation à base de paracétamol définies comme à la présente revendication 1. Elles sont caractérisées par le fait que le solvant aqueux est désoxygéné par barbotage d'un gaz inerte, qu'il est constitué par de l'eau ou par un mélange formé d'eau et d'un polyol ou d'un alcanol soluble dans l'eau, en ce que les formulations contiennent en outre un agent antiradicalaire ou capteur de radicaux libres et en ce que ledit solvant aqueux est ajusté à une valeur de pH s'échelonnant de 4 à 8 par addition d'un agent tampon.

Selon l'invention, le solvant aqueux peut être de l'eau ou bien des mélanges aqueux renfermant de l'eau et un polyol comme le polyéthylène glycol (PEG) 300, 400, 1 000, 1 540, 4 000 ou 8 000, le propylène glycol ou le tétraglycol. On peut également utiliser un alcanol soluble dans l'eau, comme par exemple l'éthanol.

La stabilité de ces solutions aqueuses n'est pas conditionnée seulement par le choix d'un véhicule. Elle est déterminée également par d'autres paramètres, comme l'ajustement judicieux du pH, l'élimination de l'oxygène dissout dans le véhicule et l'adjonction d'un agent anti-radicalaire ou capteur de radicaux libres.

L'élimination de l'oxygène dissout s'effectue commodément par barbotage d'un gaz inerte de préférence par barbotage d'azote.

L'agent anti-radicalaire approprié est choisi parmi les dérivés de l'acide ascorbique, les dérivés porteurs d'au moins une fonction thiol et les polyols linéaires ou cycliques.

Le dérivé de l'acide ascorbique est de préférence l'acide D - ou l'acide L-ascorbique, un ascorbate de métal alcalin, un ascorbate de métal alcalino-terreux ou bien encore un ester d'acide ascorbique soluble en milieu aqueux.

Le capteur de radicaux libres, porteur d'une fonction thiol peut être un composé organique substitués par une ou plusieurs fonctions thiol, de la série aliphatique comme la cystéine, l'acétytcysteine, l'acide thioglycolique et ses sels, l'acide thiolactique et ses sels, le dithiothréitol, le glutathion réduit, la thiourée, I' α-thioglycérol, la méthionine et l'acide mercaptoéthane sulfonique.

Le polyol capteur de radicaux libres est de préférence un alcool polyhydroxylé linéaire ou cyclique comme le mannitol, le sorbitol, l'inositol, l'isosorbide, le glycérol, le glucose et les propylène glycols.

Parmi les capteurs de radicaux libres dont la présence est nécessaire pour la stabilité du paracétamol, le dérivé de l'acide ascorbique actuellement préféré est l'ascorbate de sodium. Les dérivés à fonction thiol. préférés sont la cystéine, le glutathion réduit, la N-acétylcystéine et l'acide mercaptoéthane sulfonique.

II peut s'avérer avantageux d'associer plusieurs capteurs de radicaux libres dans la mesure où ils sont solubles dans l'eau et compatibles entre-eux. Un capteur de radicaux libres particulièrement avantageux est le mannitol, le glucose, le sorbitol ou encore le glycérol. Ils peuvent être associés sans difficulté.

Il peut être avantageux d'ajouter à la préparation, un agent ou plusieurs complexants pour assurer une meilleure stabilité de la molécule du fait que le principe actif est sensible à la présence de traces métalliques susceptibles de favoriser sa dégradation.

Les agents chelatants sont par exemple l'acide nitrilo triacétique, l'acide éthylène diamino tétracétique, l'acide éthylène diamino NN' -diacétique NN' -dipropionique, l'acide éthylène diamino tétra phosphonique, l'acide 2, 2' -(éthylène dimino) dibutyrique ou l'acide éthylèneglycol bis (diaminoéthyl éther) N, N, N', N' - tétracétique et leurs sels sodiques ou calciques.

Le rôle de l'agent chelatant sera également de complexer les ions divalents (Cuivre, Zinc, Cadmium) éventuellement présents qui ont une influence défavorable sur l'évolution de la forme pendant la durée du stockage.

Le gaz utilisé pour le barbotage de la solution en vue de chasser l'oxygène peut être l'azote ou le dioxyde de carbone ou encore un gaz rare. Le gaz préféré est l'azote.

L'isotonie de la préparation peut être obtenue par ajout d'une quantité judicieusement choisie de chlorure de sodium, de glucose, de lévulose ou de chlorure de potassium, ou de chlorure de calcium. ou de gluconoglucoheptonate de calcium, ou de leurs mélanges. L'agent isotonisant préféré est le chlorure de sodium.

Le tampon utilisé est un tampon compatible avec une administration injectable à l'homme, et dont le pH peut être ajusté entre 4 et 8. Les tampons préférés sont à base d'acétates ou de phosphates d'un métal alcalin ou alcalino-terreux. Le tampon davantage préféré est l'acétate de sodium, l'hydrofenophosphate ajusté au pH requis par de l'acide chlorhydrique ou de l'hydroxyde de sodium. La concentration de ce tampon peut être comprise entre 0,1 et 10 mg/ml. La concentration préférée est incluse dans les limites de 0,25 à 5 mg/ml.

Par ailleurs, les préparations injectables doivent être stériles, et doivent pouvoir être stérilisées par la chaleur. Il est connu que dans certaines conditions, des antioxydants comme le glutathion peuvent se dégrader [FIALAIRE A. et al.. J.Pharm.
Biomed. Anal.. Vol 10. N° 6. p.457-460 (1992)]. Le taux de dégradation du glutathion réduit lors d'une stérilisation par la chaleur varie de 40 à 77 % selon les conditions de température retenues. Au cours de telles stérilisations, il est donc judicieux de mettre en oeuvre les moyens susceptibles de préserver l'intégrité de ces antioxydants. L'addition de complexants à des solutions aqueuses, inhibe la dégradation par la chaleur de dérivés thiols, tels que le glutathion.

Les compositions pharmaceutiques liquides selon l'invention sont de préférence des compositions injectables. La concentration en paracétamol de la solution peut être comprise entre 2 mg/ml et 50 mg/ml s'il s'agit de solutions dites « diluées », c'est-à-dire directement prêtes à être perfusées par vois intraveineuse et entre 60 mg/ml et 350 mg/ml s'il s'agit de solutions dites « concentrées », c'est-à-dire soit destinées à être injectées directement par voie intraveineuse ou par voie intramusculaire, soit destinées à être diluées avant de les administrer en perfusion lente. Les concentrations préférées sont comprises en 5 et 20 mg/ml pour les solutions diluées et entre 100 et 250 mg/ml pour les solutions concentrées.

Les compositions pharmaceutiques selon l'invention peuvent en outre renfermer un autre principe actif qui renforce l'effet propre du paracétamol.

En particulier les compositions pharmaceutiques selon l'invention peuvent renfermer un antalgique central comme par exemple un analgésique morphinique.

L'analgésique morphinique est sélectionné parmi les dérivés morphiniques d'extraction, d'hémi-synthèse ou de synthèse et les dérivés pipéridiniques choisis dans la liste suivante, sans que celle-ci soit exhaustive : buprénorphine, ciramadol, codéine, dextromoramide, dextropropoxyphène, hydrocodone, hydromorphone, kétobémidone, lévométhadone, lévorphanol, meptazinol, méthadone, morphine, nalbuphine, nicomorphine, dizocine, diamorphine, dihydrocodéine, dipipanone, méthorphane, dextrométhorphan...

Les dérivés morphiniques préférés sont le sulfate de codéine ou le chlorhydrate de morphine.

La concentration de codéine ou du dérivé de la codéine, exprimée en codéine base, est comprise entre 0.2 et 25 % de celle du paracétamol. Le dérivé de la codéine préféré est le sulfate de codéine. Sa concentration préférée est fixée entre 0,5 et 15% de celle du paracétamol.
La concentration en morphine ou en dérivé de la morphine, exprimée en morphine base, est comprise entre 0,05 et 5 % de celle du paracétamol. Le dérivé de la morphine préféré est le chlorhydrate de morphine. Sa concentration préférée est fixée entre 0,5 et 15 % de celle du paracétamol.

Les compositions selon l'invention peuvent également être additionnées d'un agent anti-inflammatoire du type AINS et en particulier dérivé d'un acide phénylcétique. Un exemple de tels agents est le kétoprofène, le flurbiprofène, l'acide tiaprofénique, l'acide niflumique, le diclofénac ou le naproxène.

Les compositions selon l'invention peuvent également être additionnées d'un agent anti-émétique soit neuroleptique d'action centrale tel que l'haloperidol ou la chlorpromazine ou la métopimazine ou d'action gastrokinetique comme le métochlopramide ou la dompéridone ou encore un agent serotoninergique.

Les compositions selon l'invention peuvent également être additionnées d'un médicament anti-épileptique comme le valproate de sodium, le chlonazépam, la carbamazépine ou la phénytoïne.

On peut également associer au paracétamol un corticostéroïde comme par exemple la prednisone, la prednisolone, la métyl prednisone, la dexaméthasone, la bétamétasone ou un de leurs esters.

On peut également associer au paracétamol un antidépresseur tricyclique comme l'amitriptiline, l'imipramine, la chlomipramine.

Les concentrations en agents anti-inflammatoires peuvent s'échelonner de 0,100 g à 0.500 g pour 1.000 ml de préparation.

### Pour les solutions concentrées

La quantité d'eau utilisée en pourcentage est de préférence supérieure à 5% du volume final et de préférence comprise entre 10 et 65%.
La quantité de propylèneglycol utilisée en pourcentage est de préférence supérieure à 5% et de préférence comprise entre 20 et 50%.

Le PEG utilisé est de préférence le PEG 300. le PEG 400, le PEG 1000, le PEG 1540 ou le PEG 4000. Les concentrations utilisées sont comprises entre 10 et 60% en poids. Le PEG 300 et le PEG 400 sont davantage préférés. Les concentrations préférées vont de 20 à 60%.

Les concentrations d'éthanol vont de 0 à 30% du volume final et de préférence vont de 0 à 20%.
Les concentrations de tétraglycol utilisées n'excèdent pas 15% afin de tenir compte des quantités maximales administrables quotidiennement par voie parentérale, à savoir 0,7 ml/kg de poids corporel.

La concentration en glycérol varie de 0,5 à 5 % en fonction de la viscosité du milieu compatible vu le mode d'administration.

### Pour les solutions diluées

La quantité d'eau utilisée en pourcentage est de préférence supérieure à 20% du volume final et de préférence comprise entre 25 et 100%.
La quantité de propylèneglycol utilisée est en pourcentage de préférence comprise entre 0 et 10%.

Le PEG utilisé est de préférence le PEG 300, le PEG 400 ou le PEG 4000. Le PEG 4000 est préféré.

Les concentrations préférées vont de 0 à 10%.
Les concentrations de tétraglycol utilisées n'excèdent pas 5%. Elles sont comprises de préférence entre 0 et 4%.

La concentration d'acide ascorbique ou de dérivé d'acide ascorbique qui est utilisée est de préférence supérieure à 0.05 mg/ml et d'une manière davantage préférée, comprise entre 0,15 mg/ml et 5 mg/ml. Des quantités supérieures peuvent être utilisées en effet, dans les limites de la solubilité. Des doses d'acide ascorbique ou de dérivé d'acide ascorbique plus élevées sont administrées à titre préventif ou curatif à l'homme.

La concentration en dérivé thiol est comprise entre 0,001% et 30% et d'une manière davantage préférée, comprise entre 0,005% et 0,5% pour les solutions diluées, et entre 0,1% et 20% pour les solutions concentrées.

Le pH de la solution est ajusté de préférence en tenant compte de l'optimum de stabilité du paracétamol en solution aqueuse, c'est-à-dire à un pH voisin de 6,0.

La composition ainsi préparée pourra être conditionnée en ampoules de verre scellées, ou en flacons de verre bouchés ou en flacons d'un polymère tel que le polyéthylène, ou en poches souples de polyéthylène, de polychlorure de vinyle ou de polypropylène.

La composition pourra être stérilisée par traitement thermique, par exemple à 121°C pendant 20 minutes ou bien par filtration stérilisante.

Les compositions actuellement préférées selon l'invention ont les compositions suivantes :

| **Solutions concentrées** | | | |
|---|---|---|---|
| Constituant | Solution injectable de paracétamol seul (par ml) | Solution injectable de paracétamol associé à un morphinique (par ml) | |
| | | Codéine | Morphine |
| Paracétamol | 0.160 g | 0,160g | 0,160 g |
| Sulfate de codéine 3 H₂O | - | 0,0036 g | - |
| Chlorhydrate de morphine 3 H₂O | - | - | 0,00037 g |
| Propylène glycol | 0,270 ml | 0,270 ml | 0,270 ml |
| PEG 400 | 0,360 ml | 0,360 ml | 0,360 ml |
| Acétate de sodium | 0,002 g | 0,002 g | 0,002 g |
| Glutathion réduit | 0,002 g | 0,002 g | 0,002 g |
| Acide chlorhydrique 1N | qsp pH 6,0* | qsp pH 6,0* | qsp pH 6,0* |
| Eau pour préparations injectables | qsp 1,000 ml | qsp 1,000 ml | qsp 1,000 ml |
| Azote | qsp barbotage | qsp barbotage | qsp barbotage |

| | | | |
|---|---|---|---|
| * le pH indiqué est un pH réel. Il est obtenu par pHmétrie après dilution au 1/5 de la solution par de l'eau distillée. Le pH apparent de la solution pure est différent; | | | |

Cette solution composée d'un mélange solvant constitué de 30% de propylèneglycol, de 40% de polyéthylèneglycol 400 et de 30% d'eau (solution n°20), permet de solubiliser environ 200 mg/ml de paracétamol à 20°C. Le choix d'une concentration de 160 mg/ml permet d'éviter tout risque de recristallisation, notamment à basse température. Dans ces conditions, un volume de 6,25 ml de ladite solution renferme 1000 mg de paracétamol.

| **Solutions diluées** | | | |
|---|---|---|---|
| Constituant | Solution paracétamol seul (par ml) | Solution de paracétamol associé à la codéine (par ml) | |
| | | Ce morphinique est la codéine | Ce morphinique est la morphine |
| Paracétamol | 0,0125 g | 0,125 g | 0,125 g |
| Sulfate de codéine3H₂O | - | 0,00018 g | - |
| Chlorhydrate de morphine 3 H₂O | - | - | 0,000019 g |
| Mannitol | 0,025 g | 0,025 g | 0,025 g |
| Hydrogénophosphate de sodium dihydraté | 0,00025g | 0,00025g | 0,00025g |
| Chlorure de sodium | 0,0020 g | 0,0020 g | 0,0020 g |
| Ethylène diamino tétraacétate disodique | 0,0001 g | 0,0001 g | 0,0001 g |
| Acide chlorhydrique ou hydroxyde de sodium | qsp pH 5,5 | qsp pH 5,5 | qsp pH 5,5 |
| Eau pour préparations injectables | qsp 1,000 ml | qsp 1,000 ml | qsp 1,000 ml |
| Azote | qsp barbotage | qsp barbotage | qsp barbotage |

Les compositions selon l'invention trouvent leur emploi en thérapeutique comme médicament de la douleur. Pour les douleurs modérées, les solutions contiennent seulement du paracétamol. Pour les douleurs plus aiguës, les solutions contiennent, en outre, un analgésique morphinique. Par ailleurs, les solutions de paracétamol ont des propriétés antipyrétiques.

Les exemples suivants illustrent l'invention sans toutefois la limiter :

### EXEMPLE 1

### Détermination du mélange solvant optimal

### 1.1 - Solutions concentrées

Des quantités croissantes de paracétamol ont été introduites dans des mélanges de solvants. La vitesse de dissolution du paracétamol augmentant avec la température, les essais de solubilisation dans ces différents milieux ont été réalisés en chauffant à 60°C le mélange de solvants. Après dissolution complète du paracétamol, les solutions ont été placées 72 heures à 25°C et à 4°C.

Les solubilités obtenus sont rassemblées dans le tableau ci-après :

| N° essai | Eau (ml) | Propylène glycol (ml) | PEG 400 (ml) | Ethanol (ml) | Tétraglycol (ml) | Solubilité à +4°C (mg/ml) | Solubilité à +25°C (mg/ml) |
|---|---|---|---|---|---|---|---|
| 1 | 0,3 | 0.4 | 0,3 | - | - | 110 | 130 |
| 2 | 0,4 | 0,3 | 0,3 | - | - | 110 | 130 |
| 3 | 0,15 | 0,3 | 0,4 | - | 0,15 | 190 | 230 |
| 4 | 0.5 | - | 0.5 | - | - | 110 | 150 |
| 5 | 0,4 | 0,3 | 0,2 | 0,1 | - | < 110 | 120 |
| 6 | 0,5 | 0,3 | 0,1 | 0,1 | - | < 100 | 130 |
| 7 | 0,4 | 0,4 | 0,1 | 0,1 | - | < 100 | 150 |
| 8 | 0,5 | 0,3 | 0,2 | - | - | < 100 | 120 |
| 9 | 0,6 | 0,3 | 0,1 | - | - | < 100 | < 100 |
| 10 | 0,5 | 0,4 | 0.1 | - | - | < 100 | 110 |
| 11 | 0,55 | 0,3 | 0,05 | 0,1 | - | < 100 | < 100 |
| 12 | 0,45 | 0,4 | 0,05 | 0,1 | - | < 100 | 120 |
| 13 | 0,65 | 0,3 | 0,05 | - | - | < 100 | < 100 |
| 14 | 0,55 | 0,4 | 0,05 | - | - | < 100 | < 100 |
| 15 | 0,4 | 0,4 | 0,2 | - | - | < 100 | 150 |
| 16 | 0,45 | 0,45 | 0,1 | - | - | < 100 | 110 |
| 17 | 0.4 | 0,2 | 0.4 | - | - | 160 | 200 |
| 18 | 0,5 | 0,2 | 0,3 | - | - | 100 | 160 |
| 19 | 0,5 | 0,1 | 0,3 | 0,1 | - | 100 | 190 |
| 20 | 0,3 | 0,3 | 0,4 | - | - | 190 | 200 |
| 21 | 0,3 | 0,2 | 0,35 | - | 0,15 | 160 | 210 |
| 22 | 0,25 | 0,25 | 0,35 | - | 0,15 | 170 | 220 |

La solubilité dans les mélanges de solvants n'augmente pas toujours avec la température. L'adjonction d'éthanol n'augmente pas la solubilité.

En outre, en raison des phénomènes de sursaturation qui apparaissent dans de telles solutions, notamment dans les milieux contenant du PEG, on observe un retard à la cristallisation après refroidissement. Dans ces conditions, ces solutions ont été maintenues pendant 14 jours à 20°C, puis on a ajouté, dans les solutions ne présentant pas de cristaux après cette période, un cristal de paracétamol afin de provoquer la cristallisation des solutions en sursaturation éventuelle. Finalement c'est la solution n° 20 ou la solution n° 3 qui a présenté la solubilité la plus élevée en paracétamol, comprise entre 160 mg/ml et 170 mg/ml selon la température.

### 1.2 - Solutions diluées

Des quantités de paracétamol très supérieures à la limite de solubilité ont été introduites dans des mélanges de solvants portés à 30° C. Après agitation et refroidissement à 20°C, les solutions sont filtrées. La teneur de ces solutions en paracétamot est déterminée par mesure de l'absorbance à 240 nm d'une dilution au 1/200ème du filtrat.

Les résultats figurent dans les tableaux ci-après.

| Nature de la solution (sauf indication contraire, le solvant principal est l'eau distillée) | Concentration en paracétamol (mg/50 ml) |
|---|---|
| Eau | 720 |
| Glucose 5 % | 710 |
| Lévulose 4,82 % | 730 |
| Mannitol 7 % | 680 |
| Sorbitol 5 % | 685 |
| Chlorure de sodium 0,9 % | 615 |
| Gluconoglucoheptonate de calcium 10 % | 670 |
| Solution de Lestradet (glucose 5%, chlorure de sodium 0,2%, chlorure de potassium 0,15%, glucono glucoheptonate de calcium 1,1%) | 730 |
| Solution de Ringer (chlorure de sodium 0,7%, chlorure de potassium 0.1%, chlorure de sodium 0,013%) | 730 |
| Solution de Ringer phosphate (chlorure de sodium 0,7%, phosphate monopotassique 0,182%, chlorure de calcium 0,013%) | 710 |
| Solution de Ringer acétate (chlorure de sodium 0,7%, acétate de potassium 0,131%, chlorure de calcium 0,013%) | 715 |
| Urée 0,3 molaire | 725 |

| Nature de la solution (les solutions suivantes ont été réalisées dans la solution de Ringer) | Concentration en paracétamol (mg/50 ml) |
|---|---|
| Solution de Ringer pure | 735 |
| + PEG 4000 4,0% + Propylèneglycol 1,0% + Ethanol 0,5 % | 905 |
| + PEG 4000 4,0% + Propylèneglycol 1,0% + Ethanol 1,0 % | 905 |
| + PEG 4000 4,0% + Propylèneglycol 1,0% + Ethanol 2,0 % | 930 |

| Nature de la solution (les solutions suivantes ont été préparées dans une solution de chlorure de sodium 0,9 %) | Concentration en paracétamol (mg/50 ml) |
|---|---|
| Chlorure de sodium 0,9 % | 615 |
| + Tétraglycol 0,6 % | 640 |
| + Tétraglycol 1,2 % | 680 |
| + Tétraglycol 3,0 % | 720 |
| + PEG 4000 1,0 % | 630 |
| + PEG 4000 1,0% + Tétraglycol 0,6 % | 660 |
| + PEG 4000 1,0% + Tétraglycol 1.2 % | 710 |
| + PEG 4000 3,0% + Tétraglycol 2,0 % | 950 |

La présence de PEG augmente la solubilité du paracétamol.

On a déterminé les solubilités du paracétamol dans des mélanges de PEG 4000 et de solution de chlorure de sodium à 0.9% dans l'eau distillée, à des concentrations variant entre 0 et 7%, en fonction de la température.

Les résultats figurent dans le tableau suivant :

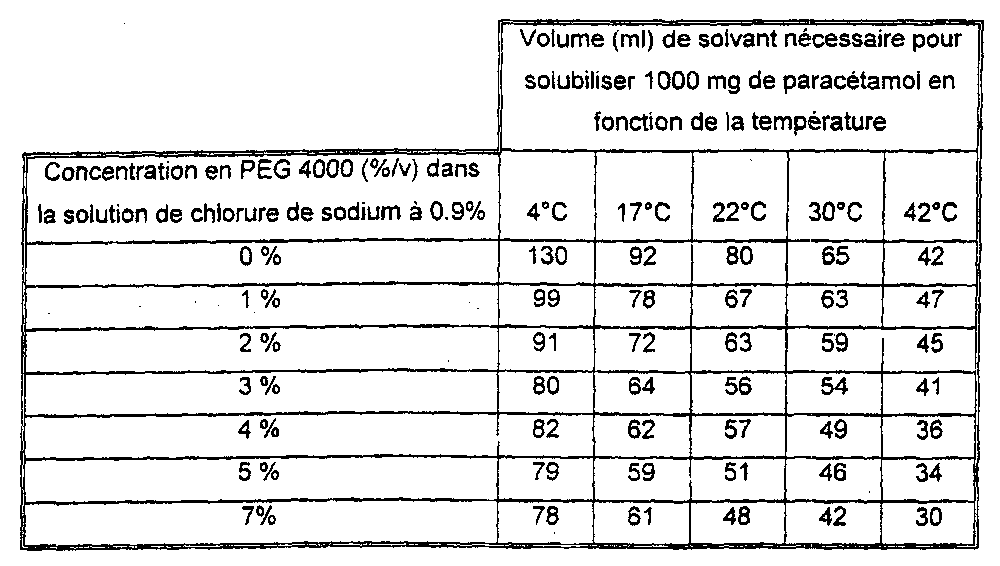

### 4.1 - Solution concentrée

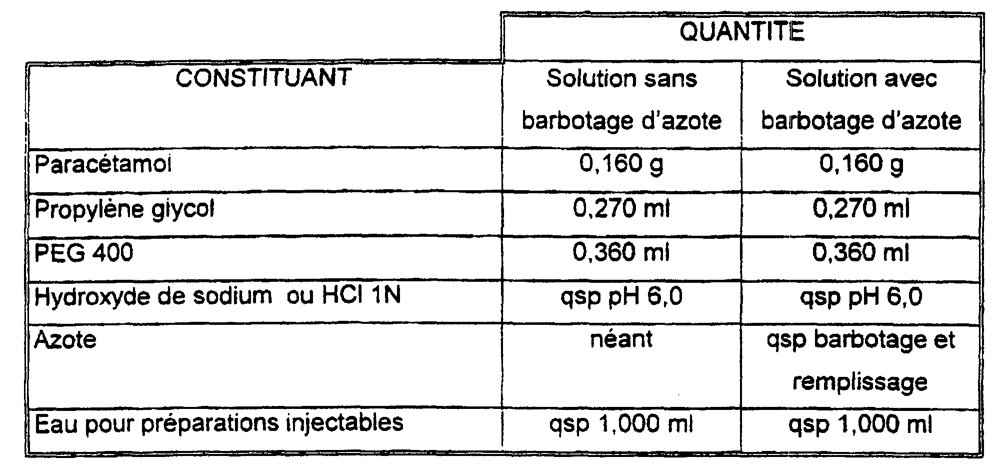

La solution 20 contenant le paracétamol à raison de 160 mg/ml, ajustée à pH 6,0 par l'hydroxyde de sodium ou l'acide chlorhydrique 1N, a subi ou non un barbotage d'azote. Des flacons remplis sous azote ou sous air, à raison de 10 ml de ces solutions, soigneusement bouchés et sertis, ont été stérilisés par autoclavage à 121°C pendant 20 minutes. On a mesuré ensuite, par chromatographie liquide, le pourcentage de pics secondaires par rapport au pic principal du paracétamol, ainsi que l'intensité de la coloration rose par mesure de l'absorbance de la solution par spectrophotométrie d'absorption à la longueur d'onde maximale d'absorption, soit 500 nm.

### Résultats

| Solution testée | Pics secondaires en % du pic principal du paracétamol | Absorbance de la solution à 500 nm |
|---|---|---|
| Solution autoclavée sans azote | 0,054 | 0,08 |
| Solution autoclavée avec azote | 0,036 | 0,03 |

La différence de coloration de la solution sous azote est donc très nette.

Afin de vérifier que les solutions de paracétamol à 0% et 1% de PEG restaient limpides au froid, les solutions suivantes ont été réalisées :

| Constituant | Solution sans PEG | Solution avec PEG 1% |
|---|---|---|
| Paracetamol | 1 g | 1 g |
| PEG 4000 | - | 1 g |
| Solution de chlorure de sodium à 0,9% dans l'eau ppi | qsp125 ml | qsp 100 ml |

Après maintien de ces solutions à 4°C pendant 10 jours, aucun des flacons testés ne présentait de cristallisation. La présence du PEG n'est donc pas nécessaire pour le maintien de la clarté de la solution dans le laps de temps étudié.

### EXEMPLE II

### Essais de détermination de la nature de la décomposition du paracétamol en solution

### 2.1 - Mise en évidence de l'instabilité du paracétamol en solution

Une solution de paracétamol dans l'eau ou dans la solution n°20 se colore rapidement en rose par exposition à la lumière ou par maintien à température élevée. A 50°C, cette coloration se produit après 2 semaines. L'application de cette coloration se traduit par une augmentation de l'absorbance de la solution à un max de 500 nm. Selon l'article de FAIRBROTHER cité plus haut, l'exposition du paracétamol à l'humidité peut conduire à une hydrolyse en para-aminophénol, suivie d'une oxydation, avec apparition d'une coloration rose, caractéristique de la formation de quinoneimine.

### 2.2 - Nature des produits de dégradation du paracétamol

Dans les solutions aqueuses ou partiellement aqueuses, on ne retrouve pas de p-aminophénol au cours de la conservation. Il se forme rapidement des composés colorés de teinte rosâtre, la vitesse de réaction étant fonction de la température et de la lumière. Au cours du temps, l'intensité de la coloration de ces dérivés augmente et évolue vers le brun.

Tout se passe donc comme si contrairement à ce qui est indiqué dans la littérature, la dégradation du paracétamol faisait d'abord appel à un processus oxydatif puis à une hydrolyse. Dans cette hypothèse. le paracétamol pourrait réagir avec un oxydant contenu dans la solution, par exemple l'oxygène dissous dans la phase aqueuse. Ce mécanisme mettrait en jeu la formation de radicaux libres permettant des couplages moléculaires, responsables de la formation de dérivés colorés évoluant du rose au brun.

### 2.3 - Essais d'inhibition de la formation de composés radicalaires

Une réaction typique mettant en jeu la formation de radicaux libres est constituée par l'addition d'une solution aqueuse d'eau oxygénée à 30 % et de sulfate de cuivre pentahydraté à 62,5 mg/ml à une solution aqueuse de paracétamol à 1,25 %. En quelques minutes, il se produit une réaction colorée évoluant du jaune au brun foncé. L'intensité de la coloration obtenue décroit si l'on ajoute préalablement à la solution de paracétamol des capteurs de radicaux libres ou du glycérol. L'intensité de la coloration est fonction de la nature du capteur de radicaux libre ajouté, dans l'ordre d'intensité décroissante suivant :
Paracétamol seul > paracétamol + N-acétylcystéine > paracétamol + cystéine > paracétamol + sorbitol > paracétamol + mannitol > paracétamol + glycérol.

### EXEMPLE III

### Stabilisation du paracétamol en solution par choix du pH de stabilité optimale

### 3.1 - Solution concentrée

| **Solution testée** | |
|---|---|
| CONSTITUANT | QUANTITE |
| Paracétamol | 0, 160 g |
| Propylène glycol | 0,270 ml |
| PEG 400 | 0,360 ml |
| Hydroxyde de sodium 1N | pH 7,0 - 8,0 - 8,5 - 9,0 - 9,5 - 10,0 |
| ou Acide chlorhydrique 1 N | correspondant à pH réel : pH 5,8 - 6,7 - |
| qsp | 7,1 - 7,5 - 8,0 - 8,5 |
| Azote qsp | barbotage et remplissage |
| Eau pour préparations injectables | qsp 1,000 ml |

La solution 20 contenant le paracétamol à raison de 160 mg/ml a été ajustée à différents pH : pH apparent au pH réel après dilution au 1/5 (entre parenthèses) : 7,0 (5.8) - 8,0 (6.7) - 8,5 (7.1) - 9,0 (7,5) - 9,5 (8,0) - 10,0 (8.5) par une solution d'hydroxyde de sodium ou d'acide chlorhydrique normale. Des flacons remplis sous azote à raison de 10 ml de ces solutions, soigneusement bouchés et sertis ont été stérilisés par autoclavage à 121°C pendant 20 minutes, puis dans tous les cas, exposés soit à 105°C à l'obscurité pendant 72 heures, soit au rayonnement d'une lumière actinique à 5000°K à 25°C pendant 264 heures.

### Résultats

Après autoclavage, seule la solution ajustée à pH 10 présente une coloration rose. Après conservation à 105°C pendant 72 heures, l'absorbance à 500 nm ainsi que la teneur en produits de dégradation du paracétamol est minimale dans la gamme de pH comprise entre 7,0 et 9,5. Après conservation à la lumière, l'intensité de la coloration croît avec le pH. Elle est minimale à pH 7,0 (réel 5,8). Ni la teneur en paracétamol, ni le taux de produits de dégradation ne sont affectés par le pH.

### 3.2 - Solution diluée

| **Solution testée** | |
|---|---|
| CONSTITUANT | QUANTITE |
| Paracétamol | 0,008 g |
| Chlorure de sodium | 0,0067 g |
| Phosphate disodique dihydraté | 0,0012 g |
| Acide citrique à 5 % qsp | pH 5,0 - 6,0 - 7,0 |
| Azote qsp | barbotage et remplissage |
| Eau pour préparations injectables | qsp 1,000 ml |

La solution aqueuse diluée et tamponnée contenant le paracétamol à raison de 8 mg/ml a été ajustée à différents pH : pH 5,0 - 6,0 - 7,0 à l'aide d'une solution d'acide citrique.

Des flacons remplis sous azote à raison de 10 ml de ces solutions, soigneusement bouchés et sertis, ont été stérilisés ou non, par autoclavage à 121°C pendant 20 minutes, puis dans tous les cas, exposés à 70°C à l'obscurité pendant 231 heures.

### Résultats

Après autoclavage, seule la solution ajustée à pH 7 présente une coloration rose. Après conservation, la même solution présente la coloration rose la plus intense. A pH 6,0 et 5,0, les solutions sont faiblement colorées.

### EXEMPLE IV

### Stabilisation du paracétamol en solution par élimination de l'oxygène par barbotage d'azote

### 4.2 - Solution diluée

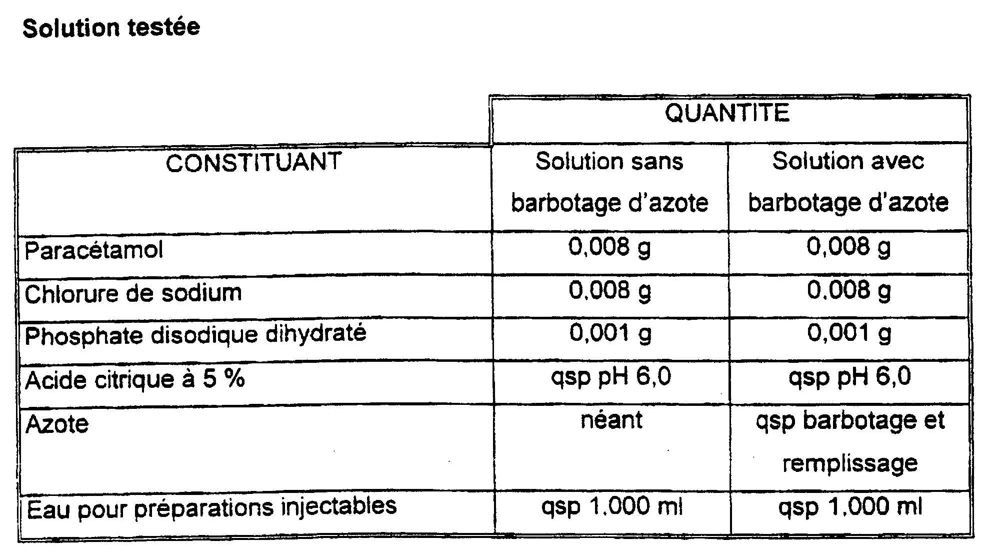

La solution aqueuse diluée contenant le paracétamol est ajustée à pH 6,0 à l'aide d'une solution d'acide citrique.

Des flacons remplis sous azote à raison de 10 ml de ces solutions, soigneusement bouchés et sertis sont maintenus à l'étuve à 98°C pendant 15 heures.
On mesure ensuite, par chromatographie liquide, le pourcentage des pics secondaires par rapport au pic principal du paracétamol, ainsi que l'intensité de la coloration rose par mesure de l'absorbance de la solution par spectrophotométrie d'absorption à la longueur d'onde maximale d'absorption, soit 500 nm.

| **Résultats** | | |
|---|---|---|
| Solution testée | Pics secondaires en % du pic principal du paracétamol | Absorbance de la solution à 500 nm |
| Solution conditionnée sans azote | 1,57 | 0,036 |
| Solution conditionnée avec azote | 0,44 | 0,016 |

La coloration rose de la solution conditionnée sous azote est considérablement plus faible que celle obtenue après stérilisation sous azote de la solution conditionnée sans azote.

### EXEMPLE V

### Stabilisation de solutions de paracétamol par addition d'agents anti-radicalaires

### 5.1 - Solution concentrée

| CONSTITUANT | QUANTITE |
|---|---|
| Paracétamol | 0,160 g |
| Propylène glycol | 0,270 ml |
| PEG 400 | 0,360 ml |
| Acide chlorhydrique 1N ou Na-OH 1N qsp | pH 6,0 |
| Capteur de radicaux libres (voir # résultats) | qs (voir # résultats) |
| Azote qsp | barbotage et remplissage |
| Eau pour préparations injectables | qsp 1,000 ml |

Les solutions ainsi préparées sont réparties en flacons de 10 ml, bouchés à l'aide d'un bouchon en Bromobutyl et operculés par une capsule aluminium. Après autoclavage à 121°C pendant 20 minutes, les flacons ont été conservés 48 heures, soit sous une lumière actinique à 5500°K à température ambiante, soit à 70°C à l'obscurité. On examine l'apparition d'une coloration éventuelle de la préparation.

| **Résultats** | | | | | |
|---|---|---|---|---|---|
| Capteur de radicaux libres | Concentration | Aspect de la Aspect de la solution à la lumière solution à 70°C | | | |
| | | Couleur | intensité | Couleur | intensité |
| Pas de capteur | - | rose | (+) | rose | (++) |
| Disulfite de sodium | 0,295 mg/ml | incolore | | incolore | |
| Ascorbate de sodium | 1,0 mg/ml | jaune | (+) | jaune | (+) |
| Glutathion réduit | 1 mg/ml | incolore | | incolore | |
| Glutathion réduit | 8 mg/ml | incolore | | incolore | |
| Cystéine chlorhydra | 1 mg/ml | trouble | | trouble | |
| α-monothioglycérol | 1 mg/ml | incolore | | incolore | |
| Dithiothréitol | 1 mg/ml | incolore | | incolore | |
| Mannitol | 50 mg/ml | incolore | | incolore | |

### 5.2 - Solution diluée

| **Solutions testées** | | | |
|---|---|---|---|
| CONSTITUANT | QUANTITE | | |
| | formulation A | formulation B | formulation C |
| Paracétamol | 0,008 g | 0,01 g 9 | 0,0125 g |
| Chlorure de sodium | 0,008 g | 0,008 g | 0,00486 g |
| Phosphate disodique dihydraté ou acétate de sodium | 0,001 g | 0,001 g | 0,00125 g |
| Acide chlorhydrique | qsp pH 6,0 | qsp pH 6,0 | qsp pH 5,5 |
| C.R.L. | qs (voir # résultats) | | |
| Azote qsp | barbotage et remplissage | | |
| Eau pour préparations injectables | qsp 1,000 ml | | |

Les solutions ainsi préparées ont été réparties en flacons de 10 ml, 100 ml ou 80 ml, bouchés à l'aide d'un bouchon en Bromobutyl et operculés par une capsule aluminium. On a examiné le rosissement éventuel de la préparation.

Après autoclavage à 121°C pendant 20 minutes, les flacons ont été conservés 48 heures, soit sous une lumière actinique à 5500°K à température ambiante, soit à 70°C à l'obscurité (formulation A).

Après autoclavage à 124°C pendant 7 minutes, les flacons ont été conservés pendant 48 heures à température ambiante à l'obscurité (formulation B et C). On a examiné le rosissement éventuel de la préparation et on a dosé le paracétamol ainsi que le C.R.L. lorsqu'il s'agissait d'un dérivé thiol.

| **Résultats** | | | | | |
|---|---|---|---|---|---|
| C.R.L. utilisé | Concentration | Aspect de la solution à la lumière | | Aspect de la solution à 70°C | |
| | | Couleur | intensité | Couleur | intensité |
| Pas de C.R.L. | - | rose | (+) | rose | (++) |
| Thio-urée | 0,5 mg/ml | incolore | | incolore | |
| Dithiothréitol | 1 mg/ml | incolore | | incolore | |
| α-monothioglycérol | 1 mg/ml | incolore | | incolore | |
| Glutathion | 1 mg/ml | incolore | | incolore | |
| Ascorbate de sodium | 0,2 mg/ml | rose | (+) | rose | (+) |
| | 0,4 mg/ml | incolore | | jaune | (+) |
| | 0,6 mg/ml | rose (+) | | jaune | (+) |
| | 1,0 mg/ml | incolore | | jaune | (+) |
| Cystéine chlorhydrate | 0,05 mg/ml | incolore | | incolore | |
| | 0,1 mg/ml | incolore | | incolore | |
| | 0,25 mg/ml | incolore | | incolore | |
| | 0,5 mg/ml | incolore | | incolore | |
| | 0,75 mg/ml | incolore | | incolore | |
| | 1 mg/ml | incolore | | incolore | |
| | 2 mg/ml | incolore | | incolore | |
| | 5 mg/ml | incolore | | incolore | |

| C.R.L. utilisé | Concentration | Aspect de la solution | | Dosages (en % de la théorie) | |
|---|---|---|---|---|---|
| | | Couleur | intensité | C.R.L. | Paracétamol |
| Cystéine chlorhydrate monohydrate | 0.2 mg/ml | incolore | | 80 % | 99.2 % |
| Cystéine chlorhydrate monohydrate | 0,5 mg/ml | incolore | | 95 % | 99,6 % |
| N-acétylcystéine | 0,2 mg/ml | incolore | | 88 % | 99,2 % |
| Mannitol | 20 mg/ml | incolore | | | |
| Mannitol | 40 mg/ml | incolore | | | |
| Mannitol | 50 mg/ml | incolore | | | |
| Glucose | 50 mg/ml | incolore | | | |

### EXEMPLE VI

### Stabilisation de solutions de paracétamol contenant un dérivé morphinique par l'addition de capteur de radicaux libres

### 6.1 - Solution concentrée

| **Solutions testées** | |
|---|---|
| CONSTITUANT | QUANTITE |
| Paracétamol | 0,160 g |
| Phosphate de codéine | 0,008 g |
| Propylène glycol | 0,270 ml |
| PEG 400 | 0,360 ml |
| Acide chlorhydrique 1 N qsp | qsp pH 6,0 |
| Capteur de radicaux libres | qs (voir # résultats) |
| Eau pour préparations injectables | qsp 1,000 ml |

Les solutions ainsi préparées sont réparties en flacons de 10 ml, bouchés à l'aide d'un bouchon en Bromobutyl et operculés par une capsule aluminium. Après autoclavage à 121°C pendant 20 minutes, les flacons ont été conservés 48 heures, soit sous une lumière actinique à 5500°K à température ambiante, soit sous une lumière actinique à 5500°K à température ambiante, soit à 70°C à l'obscurité. On examine l'apparition d'une éventuelle coloration de la préparation.

| **Résultats** | | | |
|---|---|---|---|
| Capteur de radicaux libres | Concentration | Aspect de la solution à la lumière | Aspect de la solution à 70°C |
| | | Couleur intensité | Couleur intensité |
| Pas de capteur de radicaux libres | - | rose (+) | rose (++) |
| Disulfite de sodium | 0,295 mg/ml | jaune (+) | jaune (++) |
| Ascorbate de sodium | 1,0 mg/ml | jaune (++) | jaune (+++) |
| Gluthation réduit | 1 mg/ml | jaune (+) | jaune caramel (+++) |
| | 8 mg/ml | incolore | jaune (++) |
| | 16 mg/ml | incolore | jaune (+) |
| Dithiothréitol | 1 mg/ml | rose violet (+++) | rose violet (++++) |
| Hypophosphite de sodium | 5 mg/ml | rose (+) | rose (++) |

### 6.2 - Solution diluée

| **Solutions testées** | |
|---|---|
| CONSTITUANT | QUANTITE |
| Paracétamol | 0,008 g |
| Phosphate de codéine | 0,0004 g |
| Chlorure de sodium | 0,008 g |
| Phosphate disodique dihydraté | 0,0015 g |
| Acide chlorhydique | qsp pH 6,0 |
| Capteur de radicaux libres | qs (voir # résultats) |
| Azote qsp | barbotage et remplissage |
| Eau pour préparations injectables | qsp 1,000 ml |

Les solutions ainsi préparées ont été réparties en flacons de 10 ml, bouchés à l'aide d'un bouchon en Bromobutyl et operculés par une capsule aluminium. Après autoclavage à 121°C pendant 20 minutes, les flacons ont été conservés 48 heures, soit sous une lumière actinique à 5500°K à température ambiante, soit à 70°C à l'obscurité. On a examiné l'apparition d'une coloration de la préparation.

Sur la solution ne contenant pas de capteur de radicaux libres et sur la solution contenant 0,5 mg/ml de chlorhydrate de cystéine comme agent anti-radicalaire, on dose le paracétamol et la codéine par chromatographie liquide à haute performance, immédiatement après autoclavage, comparativement aux mêmes solutions non autoclavées.

| **Résultats sur l'aspect des solutions** | | | |
|---|---|---|---|
| Capteur de radicaux libres | Concentration | Aspect de la solution à la lumière | Aspect de la solution à 70°C |
| | | Couleur intensité | Couleur intensité |
| Pas de capteur de radicaux libres | - | rose (+) | rose (+) |
| Disulfite de sodium | 0,295 mg/ml | incolore | incolore |
| Dithiothréitol | 0,5 mg/ml | incolore | incolore |
| Monothioglycérol | 0,5 mg/ml | gris | gris |
| Gluthation réduit | 2.0 mg/ml | incolore | incolore |
| N-acétylcystéine | 2,0 mg/ml | gris (+) | gris (+) |
| Cystéine chlorhydrate | 0,05 mg/ml | incolore | rose (+) |
| | 0,1 mg/ml | incolore | incolore |
| | 0.25 mg/ml | incolore | incolore |
| | 0,5 mg/ml | incolore | incolore |
| | 0,75 mg/ml | incolore | incolore |
| | 1,0 mg/ml | incolore | incolore |
| | 2,0 mg/ml | incolore | incolore |
| | 5,0 mg/ml | incolore | incolore |

| **Résultats sur le dosage du paracétamol et de la codéine** | | | |
|---|---|---|---|
| Solution testée | Constituant dosé | Solution non stérilisée | Après stérilisation |
| Solution sans capteur de radicaux libres | Paracétamol | 0,0078 g/ml | 0,0077 g/ml |
| | Codéine | 0,00043 g/ml | 0.00042 g/ml |
| Solution contenant 0,5 mg/ml de chlorhydrate de cystéine | Paracétamol | 0,0082 g/ml | 0,0081 g/ml |
| | Codéine | 0,00042 g/ml | 0,00042 g/ml |

On constate ainsi d'une part l'absence d'apparition d'une coloration et d'autre part une parfaite conservation des principes actifs après stérilisation à la chaleur.

### EXEMPLE VII

### Tolérance biologique de la préparation

### 7.1 - Tolérance hématologique

| **Solution testée** | |
|---|---|
| CONSTITUANT | QUANTITE |
| Paracétamol | 0,160g |
| Propylène glycol | 0,270 ml |
| PEG4OO | 0,360 mI |
| Azote qsp | barbotage et remplissage |
| Eau pour préparations injectables | qsp 1.000 ml |

Le pH de cette solution n'a pas été ajusté. Le pH apparent est de 7,6, soit un pH réel de 6,5.

Du sang total humain est incubé avec la solution testée, à volume égal. Toutes les 10 minutes, 2 ml du mélange sont prélevés et centrifugés 5 minutes à 5000 T/minute. 100 µl du surnageant sont dilués dans 1 ml d'eau distillée. L'absorbance de cette solution est déterminée contre de l'eau à 540 nm, longueur d'onde du maximum d'absorption de l'hémoglobine.

L'étude est réalisée comparativement à un témoin négatif (sérum physiologique) et un témoin positif (eau pour préparations injectables pure).

### Résultats

Les absorbances des différentes solutions après différents temps d'incubation sont fournies dans le tableau ci-après

| Solution | T0 | 10 min | 20 min | 30 min | 40 min | 50 min | 60 min |
|---|---|---|---|---|---|---|---|
| Eau p.p.i | 2.23 | 2.52 | 2,30 | 2,37 | 2,38 | 2,33 | 2,36 |
| Sérum physiol. | 0,04 | 0,05 | 0,05 | 0,05 | 0,04 | 0,05 | 0,04 |
| Sol. testée | 0,09 | 0,19 | 0,27 | 0,25 | 0,24 | 0.24 | 0,25 |

Aucun effet d'hémolyse ne peut être décelé.

### 7.2 - Tolérance musculaire

| **Solution testée** | |
|---|---|
| CONSTITUANT | QUANTITE |
| Paracétamol | 0,160g |
| Propylène glycol | 0,270 ml |
| PEG 400 | 0,360 ml |
| Azote qsp | barbotage et remplissage |
| Eau pour préparations injectables | qsp 1,000 ml |

Le pH de cette solution n'a pas été ajusté. Le pH apparent est de 7.6.

Des rats Sprague-Dawley. pesant entre 260 g et 450 g sont anesthésiés par une injection IP de carbamate d'éthyle (2 ml/kg d'une solution aqueuse à 50%). Le muscle extensor digitorum longus est prélevé de la patte arrière, gauche ou droite, et placé dans un milieu tampon répondant à la composition suivante :

| CONSTITUANT | QUANTITE |
|---|---|
| Chlorure de sodium | 6,8 g |
| Chlorure de potassium | 0,4 g |
| Dextrose | 1,0 g |
| Bicarbonate de sodium | 2,2 g |
| Rouge de phénol (sel de sodium) | 0,005 g |
| Eau distillée qsp | 1 litre |
| Acide chlorhydrique 1 N qsp | pH 7,4 |

Le muscle est provisoirement fixé sur une planchette et maintenu par les tendons. Le produit à étudier est injecté à raison de 15 µl à l'aide d'une seringue Hamilton n°702 de 25 µl de capacité. Le muscle est ensuite placé sur une grille et plongé dans la solution tampon maintenue à 37°C sous barbotage de carbogène pendant toute la durée de l'incubation. Toutes les 30 minutes, les muscles sont introduits dans un tube contenant un tampon neuf à 37°C. L'opération est recommencée 4 fois. La solution de tampon incubée est analysée pour détermination de l'activité de la créatine-kinase.

L'étude est menée comparativement à :
- muscle seul non injecté (blanc)
- aiguille seule (introduction de l'aiguille sans injection de produit)
- sérum physiologique
- solution de Triton X-100 (témoin positif)
- solution 20
- solution 20 + paracétamol 160 mg/ml

La créatine-kinase est dosée sur un automate HITACHI 704 à l'aide du kit réactif Enzyline CK NAC optimisé 10 (Biomérieux).

### Résultats

Les activités de la créatine-kinase (Ul/l) dans les différentes solutions après différents temps d'incubation sont fournies dans le tableau ci-après :

| Solution testée | 30 min | 60 min | 90 min | 120 min | TOTAL |
|---|---|---|---|---|---|
| Muscle seul | 23 ± 6 | 24 ± 12 | 15 ± 7 | 13 ± 5 | 75 |
| Aiguille seule | 35 ± 6 | 33 ± 10 | 20 ± 4 | 18 ± 7 | 106 |
| Sérum physiol. | 30 ± 6 | 30 ± 12 | 17 ± 5 | 23 ± 4 | 100 |
| Triton X-100 | 12802 ± 2114 | 1716 ± 978 | 155 ± 89 | 289 ± 251 | 14 962 |
| Solution 20 (excipients) | 71 ± 24 | 89 ± 40 | 39 ± 27 | 62 ± 39 | 261 |
| Solution 20 + paracétamol | 141 ± 40 | 150 ± 60 | 68 ± 63 | 34 ± 24 | 393 |

Aucun phénomène de nécrose ne peut être constaté avec les compositions selon l'invention, les différences entre les résultats cumulés avec la solution excipient n'étant pas significatives.

## Revendications

1. Formulations liquides, stables, à l'oxydation, à base de paracétamol dans un solvant aqueux, **caractérisées en ce que** le solvant aqueux est désoxygéné par barbotage d'un gaz inerte, qu'il est constitué par de l'eau ou par un mélange formé d'eau et d'un polyol ou d'un alcanol soluble dans l'eau, **en ce que** les formulations contiennent en outre un agent antiradicalaire ou capteur de radicaux libres et **en ce que** ledit solvant aqueux est ajusté à une valeur de pH s'échelonnant de 4 à 8 par addition d'un agent tampon.

2. Formulations liquides à base de paracétamol selon la revendication 1, dans lesquelles l'agent tampon additionné fournit un pH de l'ordre de 6,0.

3. Formulations liquides à base de paracétamol, stables, selon la revendication 1, dans lesquelles le capteur de radicaux libres est choisi parmi les dérivés de l'acide ascorbique, les composés organiques porteurs d'au moins une fonction thiol, et les polyols.

4. Formulations liquides à base de paracétamol, stables, selon la revendication 1 ou la revendication 3, dans lesquelles les dérivés de l'acide ascorbique sont choisis dans le groupe formé de l'acide D-ascorbique, de l'acide L-ascorbique, des ascorbates de métal alcalin, des ascorbates de métal alcalino-terreux et des esters d'acide ascorbique solubles en milieu aqueux.

5. Formulations liquides à base de paracétamol, stables, selon la revendication 1 dans lesquelles le composé organique, porteur d'au moins une fonction thiol, est choisi parmi les composés de la série aliphatique ou cyclanique, ayant une ou plusieurs fonctions thiol.

6. Formulations liquides à base de paracétamol, stables, selon la revendication 1 et la revendication 3, dans lesquelles le composé porteur d'une fonction thiol est choisi dans le groupe formé de l'acide thioglycolique, de l'acide thiolactique, du dithiothréitol, du glutathion réduit, de la thiourée, de l'α-thioglycérol, de la cystéine, de l'acétylcystéine et de l'acide mercaptoéthane sulfonique.

7. Formulations liquides à base de paracétamol, stables, selon la revendication 3, dans lesquelles le polyol est un alcool aliphatique polyhydroxylé ayant de 2 à 10 atomes de carbone.

8. Formulations liquides à base de paracétamol, stables, selon la revendication 3 et la revendication 7, dans lesquelles le polyol est un sucre, un glucitol, linéaire ou cyclique, ayant de 2 à 10 atomes de carbone, choisis parmi le mannitol, le sorbitol, l'inositol, le glucose et le glycérol.

9. Formulations liquides à base de paracétamol, stables, selon l'une des revendications 1 à 8, **caractérisées en ce qu'**elles renferment en outre au moins un agent complexant.

10. Formulations liquides à base de paracétamol, stables, selon l'une des revendications 1 à 9, dans lesquelles la concentration en paracétamol varie de 2 mg à 50 mg/ml pour des solutions diluées.

11. Formulations liquides à base de paracétamol, stables, selon l'une des revendications 1 à 9, dans lesquelles la concentration en paracétamol varie de 60 mg à 350 mg/ml pour des solutions concentrées.

12. Formulations liquides à base de paracétamol, stables, selon l'une des revendications 1 à 9, dans lesquelles on ajoute à la préparation une quantité calculée d'agent isotonisant.

13. Formulations liquides à base de paracétamol, stables, selon l'une des revendications 1 à 9, **caractérisées en ce que** pour l'administration par voie parentérale, on stérilise les solutions à la chaleur.

14. Formulations liquides à base de paracétamol, stables, selon l'une des revendications 1 à 9 **caractérisées en ce qu'**elles renferment en outre un antalgique central comme par exemple un analgésique morphinique.

15. Formulations liquides à base de paracétamol, stables, selon la revendication 14, dans lesquelles l'analgésique morphinique est un dérivé morphinique d'extraction, d'hèmi-synthèse ou de synthèse, un dérivé de la phénylpipéridine, un dérivé de l'acide nipécotique, un dérivé du phénylcyclohexanol ou un dérivé de la phénylazépine.

16. Formulations liquides à base de paracétamol, stables, selon la revendication 14 et la revendication 15, dans lesquelles l'analgésique morphinique est présent à une dose variant de 0,05 à 5 % du paracétamol lorsqu'il s'agit de la morphine et de 0,2 à 2,5 % lorsqu'il s'agit de la codéine.

17. Formulations liquides à base de paracétamol, stables, selon l'une des revendications 1 à 9 **caractérisées en ce qu'**elles renferment en outre un agent anti-inflammatoire du type phénylacétique.

18. Formulations liquides à base de paracétamol, stables, selon la revendication 17 **caractérisées en ce que** l'agent anti-inflammatoire est le kétoprofène.

19. Formulations liquides à base de paracétamol, stables, selon l'une des revendications 1 à 9, **caractérisées en ce qu'**elles renferment en outre un agent anti-émétique.

20. Formulations liquides à base de paracétamol, stables, selon l'une des revendications 1 à 9, **caractérisées en ce qu'**elles renferment en outre un agent anti-épileptique.

21. Formulations liquides à base de paracétamol, stables, selon l'une des revendications 1 à 9, **caractérisées en ce qu'**elles renferment en outre un corticostéroïde.

22. Formulations liquides à base de paracétamol, stables, selon l'une des revendications 1 à 9, **caractérisées en ce qu'**elles renferment en outre un agent antidépresseur tricyclique.

## Patentansprüche

1. Flüssige, gegen Oxidation stabile Rezepturen auf der Basis von Paracetamol in einem wässrigen Lösungsmittel, **dadurch gekennzeichnet, dass** das wässrige Lösungsmittel durch das Durchblasen mit einem inerten Gas, von Sauerstoff befreit ist; dass es aus Wasser oder einer Mischung besteht, die aus Wasser und Polyalkohol oder einem in Wasser löslichen Alkohol besteht; dass die Rezepturen außerdem ein antiradikalisches Mittel oder einen Fänger für freie Radikale enthalten und dass das besagte wässrige Lösungsmittel einen durch die Zugabe einer Pufferlösung angepassten pH-Wert im Bereich von 4 bis 8 aufweist.

2. Flüssige Rezepturen auf der Basis von Paracetamol gemäß Anspruch 1, bei denen die zugegebene Pufferlösung einen pH in der Größenordnung von 6,0 ergibt.

3. Flüssige, stabile Rezepturen auf der Basis von Paracetamol gemäß Anspruch 1, bei denen der Fänger für freie Radikale aus Derivaten der Ascorbinsäure, organischen Verbindungen, die wenigstens eine Thiolgruppe trägen, und Polyalkoholen ausgesucht wird.

4. Flüssige, stabile Rezepturen auf der Basis von Paracetamol gemäß Anspruch 1 oder Anspruch 3, bei denen die Derivate der Ascorbinsäure aus der Gruppe ausgewählt werden, die aus D-Ascorbinsäure, L-Ascorbinsäure, Alkalimetalle Ascorbaten, Erdalkalimetalle Ascorbaten and in wässrigem Milieu löslichen Estern der Ascorbinsäure gebildet wird.

5. Flüssige, stabile Rezepturen auf der Basis von Paracetamol gemäß Anspruch 1, bei denen die organische Verbindung, die wenigstens eine Thiolgrupppe trägt, aus den Verbindungen der aliphatischen und cyclischen Reihe ausgewählt wird, die eine oder mehrere Thiolgruppen aufweisen.

6. Flüssige, stabile Rezepturen auf der Basis von Paracetamol gemäß Anspruch 1 und Anspruch 3, bei denen die Verbindung, die wenigstens einer Thiolgrupppe tragen, aus der Gruppe ausgesucht wird, die aus Thioglykolsäure, Thiomilchsäure, Dithiothreitiol, reduziertem Gluthathion, Thioharnstoff, Alpha-Thioglycerol, Cystein, Acetylcystein, und Mercaptoethansulfonsäure gebildet wird.

7. Flüssige, stabile Rezepturen auf der Basis von Paracetamol gemäß Anspruch 3, bei denen der Polyalkohol ein aliphatischer polyhydroxylierter Alkohol mit 2 bis 10 Kohlenstoffatomen ist.

8. Flüssige, stabile Rezepturen auf der Basis von Paracetamol gemäß Anspruch 3 und Anspruch 7, bei denen der Polyalkohol ein linearer oder cyclischen Zucker, ein Glucitol, mit 2 bis 10 Kohlenstoffatomen ist, der aus Mannit, Sorbit, Inosit, Glukose und Glyzerin ausgesucht wird.

9. Flüssige, stabile Rezepturen auf der Basis von Paracetamol gemäß einem der Ansprüche 1 bis 8, **dadurch gekennzeichnet, dass** sie außerdem wenigstens einen Komplexbildner enthalten.

10. Flüssige, stabile Rezepturen auf der Basis von Paracetamol gemäß einem der Ansprüche 1 bis 9, bei denen für die verdünnten Lösungen die Konzentration von Paracetamol zwischen 2 mg und 50 mg/ml variiert.

11. Flüssige, stabile Rezepturen auf der Basis von Paracetamol gemäß einem der Ansprüche 1 bis 9, bei denen für die konzentrierten Lösungen die Konzentration von Paracetamol zwischen 60 mg und 350 mg/ml variiert.

12. Flüssige, stabile Rezepturen auf der Basis von Paracetamol gemäß einem der Ansprüche 1 bis 9, bei denen bei der Herstellung ein bestimmte Menge Isotonisierungsmittel hinzugefügt wird.

13. Flüssige, stabile Rezepturen auf der Basis von Paracetamol gemäß einem der Ansprüche 1 bis 9, **dadurch gekennzeichnet, dass** sie parenteral verabreicht werden und dass man die Lösungen durch Hitze sterilisiert.

14. Flüssige, stabile Rezepturen auf der Basis von Paracetamol gemäß einem der Ansprüche 1 bis 9, **dadurch gekennzeichnet, dass** sie außerdem ein schmerzstillendes Mittel mit zentraler Wirksamkeit, wie zum Beispiel Morphin enthaltende Analgetika, beinhalten.

15. Flüssige, stabile Rezepturen auf der Basis von Paracetamol gemäß Anspruch 14, bei denen das Morphin enthaltende Analgetika ein extrahiertes, halbsynthetisches oder synthetisches Morphinderivat, ein Derivat des Phenylpiperidin, ein Derivat der Nipecotsäure, ein Phenylcyclohexanol oder ein Derivat der Phenylazepine, ist.

16. Flüssige, stabile Rezepturen auf der Basis von Paracetamol gemäß Anspruch 14 und Anspruch 15, bei denen das Morphin enthaltende Analgetika in einer Dosis vorliegt, die zwischen 0.05 und 5% des Paracetamols für den Fall, dass es sich um Morphin handelt, und zwischen 0,2 bis 2,5% für den Fall, dass es sich um Codein handelt, variiert.

17. Flüssige, stabile Rezepturen auf der Basis von Paracetamol gemäß einem der Ansprüche 1 bis 9, **dadurch gekennzeichnet, dass** sie außerdem einen entzündungshemmenden Wirkstoff vom Typ Phenylacetat enthalten.

18. Flüssige, stabile Rezepturen auf der Basis von Paracetamol gemäß Ansprüche 17, **dadurch gekennzeichnet, dass** der entzündungshemmende Wirkstoff Ketoprofen ist.

19. Flüssige, stabile Rezepturen auf der Basis von Paracetamol gemäß einem der Ansprüche 1 bis 9, **dadurch gekennzeichnet, dass** sie ausserdem ein Mittel gegen Erbrechen enthalten.

20. Flüssige, stabile Rezepturen auf der Basis von Paracetamol gemäß einem der Ansprüche 1 bis 9, **dadurch gekennzeichnet, dass** sie außerdem ein Mittel gegen Epilepsie enthalten.

21. Flüssige, stabile Rezepturen auf der Basis von Paracetamol gemäß einem der Ansprüche 1 bis 9, **dadurch gekennzeichnet, dass** sie außerdem Corticosteroid enthalten.

22. Flüssige, stabile Rezepturen auf der Basis von Paracetamol gemäß einem der Ansprüche 1 bis 9, **dadurch gekennzeichnet, dass** sie außerdem ein tricyclisches Antidepressiva enthalten.

## Claims

1. Liquid, stable to the oxydation formulations based on paracetamol in an aqueous solvent, **characterized in that** the aqueous solvent is deoxygenated by bubbling into an inert gaz, that it is constituted by water or a mixture based on water and a polyol, or a water soluble alkanol, **in that** the formulation further contain an anti-radical agent or a free radical scavenger, and **in that** the said aqueous solvent is adjusted to a value of pH ranging from to 4 to 8, by adding a buffer agent.

2. Liquid formulations based on Paracetamol according to claim 1, wherein the buffer agent to be added provides a pH of the order of 6,0.

3. Stable liquid formulations based on paracetamol according to claim 1 wherein the free radical scavenger is selected among the derivatives of ascorbic acid, the organic compounds which bear at least one thiol function and the polyols.

4. Liquid stable formulations based on paracetamol according to claim 1 or claim 3, wherein the derivatives of ascorbic acid are selected from the group formed of D-ascorbic acid, L-ascorbic acid , alkalimetal ascorbates, earth alkaline ascorbates, and esters of ascorbic acid which are soluble in aqueous medium.

5. Liquid, stable formulations based on paracetamol according to claim 1, wherein the organic compound which bears at least one thiol function, is selected among the compounds of the aliphatic or cyclanic series having one or several thiol fonctions.

6. Liquid, stable formulations based on paracetamol, according to claim 1 and claim 3, wherein the compound which bears a thiol fonction is selected form the group formed by thioglycolic acid, thiolactic acid, dithiothreitol, reduced glutathion, thiourea, α-thioglycerol, cystein, acetylcystein, and mercaptoethane sulfonic acid.

7. Liquid stable formulations based on paracetamol according to claim 3, wherein the polyol is an aliphatic polyhydroxylated alcohol having from 2 to 10 carbon atoms.

8. Liquid, stable formulations based on paracetamol, according to claim 3 and claim 7 wherein the polyol is a sugar, a linear or cyclic glucitol having from 2 to 10 carbon atoms selected from mannitol, sorbitol, inositol, glucose and glycerol.

9. Liquid, stable formulations based on paracetamol according to one of the claims 1 to 8 **characterized in that** they further contain at least one complexing agent.

10. Liquid stable formulations based on paracetamol, according to one of the claims 1 to 9, wherein the concentration in paracetamol ranges form 2 mg to 20mg/l for the diluted solution.

11. Liquid stable formulations based on paracetamol according to one of the claims 1 to 9 wherein the concentration in paracetamol ranges from 60 to 350 mg/ml for the concentrated solutions.

12. Liquid, stable formulations based on paracetamol, according to one of the claims 1 to 9, wherein a just calculated amount of isotonizing agent is added.

13. Liquid, stable formulations based on paracetamol according to one of claims 1 to 9, **characterized in that** for administration through parenteral way, the solutions are sterilized by heating.

14. Liquid, stable formulations based on paracetamol according to one of the claims 1 to 9, **characterized in that** they further contain a central analgesic such as for example a morphinic analgesic.

15. Liquid stable formulation based on paracetamol according to claim 14, wherein the w morphinic analgesic is a morphinic derivative resulting from extraction, hemi-synthesis, or synthesis a derivative of phenylpiperidine, a derivative of nipecotic acid, a derivative of phenylcyclohexanol or a derivative of phenylazepine.

16. Liquid stable formulations based on paracetamol according to claim 14 and claim 15,
wherein the morphinic analgetic is present therein at a dosage ranging from 0,05 to 5% of that of paracetamol when it is a matter of morphine and from 0.2 to 2.5% when it is a matter of codeine.

17. Liquid, stable formulations based on paracetamol according to one of the claims 1 to 9 **characterized in that** they further contain an anti-inflammatory agent of the phenylacetic type.

18. Liquid, stable formulations based on paracetamol according to claim 17, **characterized by** the fact that the anti-inflammatory agent is ketoprofene.

19. Liquid stable formulations based on paracetamol according to one of the claims 1 to 9, **characterized in that** they further contain an anti-emetic agent.

20. Liquid, stable formulations based on paracetamol according to one of the claims 1 to 9, **characterized in that** they further contain an anti epileptic agent.

21. Liquid, stable formulations based on paracetamol according to one of the claims 1 to 9, **characterized in that** they further contain a corticosteroid.

22. Liquid, stable formulations based on paracetamol, according to one of the claims 1 to 9, **characterized in that** they further contain a trycyclic antidepressive agent.
